# Europäisches Patentamt

## European Patent Office

## Office européen des brevets

(11) Publication number: **0 010 206**
**B1**

(12) **EUROPEAN PATENT SPECIFICATION**

(45) Date of publication of patent specification: **04.05.83**

(21) Application number: **79103676.7**

(22) Date of filing: **28.09.79**

(51) Int. Cl.³: **C 07 C 51/25, C 07 C 57/04, B 01 J 8/04, B 01 J 8/24**

(54) A process for the oxidation of propylene to acrylic acid and of isobutylen to methacrylic acid.

(30) Priority: **24.10.78 US 954262**

(43) Date of publication of application:
**30.04.80 Bulletin 80/9**

(45) Publication of the grant of the patent:
**04.05.83 Bulletin 83/18**

(84) Designated Contracting States:
**BE CH DE FR GB IT LU NL SE**

(56) References cited:
FR - A - 1 161 722
FR - A - 2 080 231
GB - A - 1 019 235
US - A - 2 631 921
US - A - 2 931 770
US - A - 3 808 121
US - A - 3 907 859

(73) Proprietor: **THE STANDARD OIL COMPANY**
**Midland Building**
**Cleveland, Ohio 44115 (US)**

(72) Inventor: **Callahan, James Louis**
**RD 6 Township Road No. 101**
**Wooster, Ohio 44691 (US)**
Inventor: **Shaw, Wilfried Garside**
**1028 Linden Lane**
**Lyndhurst, Ohio 44124 (US)**
Inventor: **Miller, Arthur Francis**
**4827 S. Sedgewick Road**
**Lyndhurst, Ohio 44124 (US)**

(74) Representative: **Gille, Christian, Dipl.-Ing. et al,**
**Redies , Redies, Türk & Gille Bruckner Strasse 20**
**D-4000 Düsseldorf 13 (DE)**

Courier Press, Leamington Spa, England

## A process for the oxidation of propylene to acrylic acid and of isobutylene to methacrylic acid

In the production of acrylic acid or methacrylic acid the first step is typically the conversion of the reactants to acrolein or methacrolein in a fixed bed. This is followed by a second reactor using a fixed-bed catalyst to convert acrolein to acrylic acid and methacrolein to methacrylic acid respectively. The object of the present invention is to provide such processes which may be achieved in one reactor where both fixed and fluid-bed operations can take place. This reduces capital cost, simplifies the process, and allows the advantages of both fixed and fluid-bed operations.

Attempts to combine fixed and fluid-bed catalytic reactions in one bed have been made previously. US—PS 3,374,052 discloses a vessel with a movable top plate that closes in on the top of the fluid bed. This pressure from the plate renders the top of the bed non-fluidized, or fixed. This method, however, would be difficult to apply to a chemical reaction involving more than one type of catalyst in the same reactor, and this fixed-bed section is not interspersed throughout the fluid catalyst bed, resulting in disadvantage such as heat transfer capabilities.

US—PS 3,808,121 discloses a regenerative reactor wherein in reaction zones of one single type coke is burned off a fluid-bed catalyst. Fixed-bed catalysts within the regenerative reactor convert the products from the coke burning to carbon dioxide. There is only one catalytic reaction occurring. GB—PS 1,019,235 discloses a method of eliminating bubbles in a fluid-bed reactor by providing two types of the same catalyst. Again only one reaction is taking place. US—PS 3,907,859 discloses that two gas phase reactions may take place in a single reactor. However there are no teachings to the use of a fixed-bed catalyst within a fluid-bed.

The present invention is a process for the oxidation of propylene to acrylic acid and of isobutylene to methacrylic acid which is characterized in that the gas phase reactions of the olefin to the aldehyde and aldehyde to the acid occur within a single fluid-bed reactor having a fluid-bed catalyst suitable for converting olefins to aldehydes, and having within said fluid bed one or more fixed bed catalysts suitable for the conversion of aldehydes to acids.

The selection of the type of bed for a given reaction is dependent on several factors. Fixed-bed reactors tend to provide excellent gas solids contacting, less catalyst then for a fluid bed, and a smaller reactor. Fixed-bed reactors also allow the grading of catalyst within the reactor, and different types of catalyst may be installed at different locations. However, in reactions in which there is a large amount of heat released or absorbed, fixed-bed reactors require a large amount of heat transfer surface to adequately control the reaction temperature. The fixed-bed particles are normally much larger than fluid-bed particles, and therefore are more sensitive to reactant diffusion complications. Finally, fixed-bed reactors generally require efficient mixing of all reactants prior to entering the catalyst bed. The type of reaction to be employed in a particular operation can be determined only after considering all these features.

Fluid-bed reactors are achieved where gas is passed through the reactor at a sufficient velocity to "fluidize" the catalyst particles. Because of this fluidized state, such beds have excellent heat conductivity and require only moderate amounts of heat transfer area. The feeds do not have to be well mixed, for this may take place within the bed. However, along with increased reactor size, a fluid bed tends to bypass reactant gas due to bubble formation. Grading of the catalyst is normally not possible due to the high degree of mixing in such a bed.

The invention as claimed is intended to provide a remedy. It solves the problems normally associated with each type of bed, yet retains the advantages of both. In reactions involving several stages or steps, the present invention allows the combination of such steps in one reactor.

Central to the present invention is that the fixed-bed catalysts are located within the fluid bed. The catalyst located in the fixed bed do not move within the reactor. However, the fluid-bed particles also within the reactor move through the fixed bed in both an upward and downward direction. This movement of the fluid-bed catalyst achieves the beneficial results of good heat transfer and good mixing within the fixed catalyst bed.

The fixed-bed catalyst may be supported within the reactor on horizontal grids or screens, known in the art, with such an effective mesh size that prevents the fixed-bed particles from moving downward. As mentioned previously, fixed-bed catalysts are normally larger than fluid-bed catalysts. For the present invention, fixed-bed particles in the size range of 0.08 to 2.56 cm effective diameter are preferred. Preferably the effective diameter is from 0.08 cm to 2.54 cm.

This size allows good movement of the fluid-bed particles through the fixed-bed catalyst. While any fixed-bed particle shape may be used, for most applications particles which are approximately spherical in shape are preferred.

The support grids containing the fixed-bed catalyst may also have another grid or screen to limit the horizontal dispersement of the fixed-bed particles. Where needed, this would achieve a relatively uniform thickness of fixed-bed catalyst over the entire surface of the support grid. Without such a grid, the fixed-bed catalyst tends to build up against the reactor wall.

Even so, the catalyst pellets rearrange themselves until a natural downcomer is formed.

The depth of the fixed-bed catalyst should be no more than 60 cm for any individual bed. This limitation allows for the movement of the fluid particles with a minimum of pressure drop. Preferred is a depth of about 1,5 to about 58 cm.

The number of zones of fixed-bed catalysts may vary depending on the type of reaction. Only one bed may be used however the number of zones may increase. In such reactions it is preferred that these zones be located in the upper portion of the fluidized bed. However, these zones may be located anywhere within the reactor with the exception of the very lowermost section. Also preferred is at least 3 cm of free space between the top of the fixed-bed catalyst level of a given bed and the lower level of the fixed bed immediately above it.

The particles of the fluid-bed catalysts have a range from about 5 to about 300 $\mu$m in diameter. The size and type of fluid-bed catalysts are known in the art. In all cases, however, a zone of pure fluidized catalyst should be maintained in the lowermost section of the reactor to insure proper mixing of reactants.

The composition of the catalyst may vary. The fixed-bed catalyst may be the same composition as the fluid bed, containing one or more different catalysts. The fluid-bed catalyst may also contain different compositions than the fixed bed. It may also be a mixture of one or more catalyst compositions. Because of the invention's unique design, many variations of different catalysts may be used to optimize the present reaction.

The fluidized bed may also contain sieve trays in the pure fluid regions of the reactor. The use of such trays in fluid-bed reactors are well known.

The reactor is any type normally associated with fluid-bed reactions. Typically the reactor consists of a disengaging zone where the catalyst is separated from the products, and a catalyst zone where the reaction takes place. The location of the fixed beds can be within either zone.

The reactor may additionally contain overflow downcomers to permit fluid catalyst to circulate from the region above the uppermost level of fixed-bed catalyst to some lower elevation in the reactor. The use of such devices will provide greater flow of fluid-bed particles through the fixed-bed zones.

The use of such known in the art devices as cyclones, filters and vertical or horizontal cooling coils within the reactor are also contemplated by the present invention.

It may be advantageous to place the fixed-bed zone in the upper section of the fluidized bed at a different temperature than the rest of the reactor to help promote the desired reaction.

The catalyst circulation rate is apparently affected by the superficial linear vapor velocity. As this velocity increases, the catalyst circulation rate decreases. It has also been found that, depending upon the specific process variables, a certain amount of time is necessary for the system to stabilize and achieve a constant pressure drop through the total bed.

The invention thus achieves the desired benefits of fixed and fluid-bed operations without the disadvantages normally associated with each separately. This process improves heat transfer and reactor temperature control capabilities of fixed-bed reactors alone. The present invention allows for the elimination of one or more additional reactors, interstage lines, etc., thus greatly reducing the capital costs normally associated with the preparation of acrylic acid and methacrylic acid.

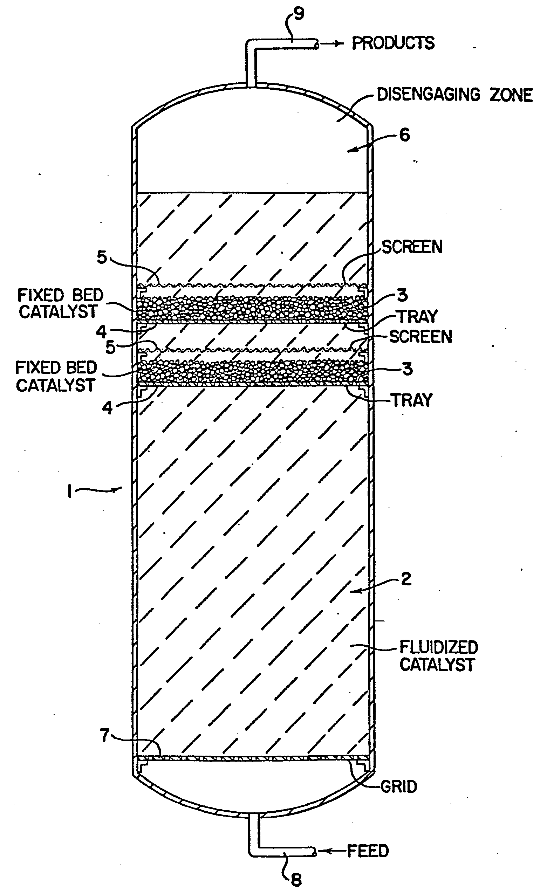

One specific embodiment of the invention is described in detail below with reference to the drawing which shows schematically a fluidized bed reactor having two fixed-bed catalyst trays.

Referring to the drawing, the fixed-fluid bed reactor 1 contains catalyst 2 in the fluid state. This fluid catalyst is supported at the bottom of the reactor by a grid 7.

Within the fluid bed are shown two trays 4. The number of trays will depend upon the amount of fixed-bed catalyst desired.

The fixed-bed catalyst 3 rests on the trays 4. Screens 5 are provided to minimize the horizontal displacement of the fixed-bed catalyst caused by the movement of the fluid-bed catalyst through the trays.

A disengaging zone 6 is provided wherein the catalyst is separated from the reaction products. The fixed-bed catalysts 3 can be located within the disengaging zone or within the catalyst zone.

Feed enters through line 8 at the bottom of the reactor. Although shown with one entry point, it is also common to provide a fluidizing feed separate from the other reactants. The feed passes through the reactor at such a velocity to fluidize the catalyst within the bed. The fluidized catalyst is in constant circulation within the reactor, passing through the fixed-bed catalyst in both directions.

Reaction of the feed takes place in both the fluidized catalyst and fixed-bed catalyst sections. Finally, the reaction products are removed from the top of the reactor above the disengaging zone through line 9. In some fluidized-bed reactors, cyclones are used at the top of the reactor to separate the fluidized-bed catalyst from the products.

Comparative Example A and Examples 1 to 3

A 3.81 cm diameter 61 cm high sieve tray reactor was used to show the advantages of the present invention with respect to acrylic acid production. Approximately 550 cc of fluid-bed catalyst was used. This catalyst consisted of two types blended together; a first catalyst, 90% of an 82% $K_{0.1}Ni_{2.5}Co_{4.5}Fe_3Bi_1P_{0.5}Mo_{12}O_{50.3}$ — 18% $SiO_2$ useful for the conversion of propylene to acrolein and a second catalyst, 10% of a 62.5% $V_3W_{1.2}Mo_{12}O_{47.1}$ — 37.5% $SiO_2$ useful for the conversion of acrolein to acrylic acid. The particle size range was 5 to 149 $\mu$m, with average particle size about 55 to 60 $\mu$m.

For Examples 1 to 3 a fixed-bed catalyst of 20% $Cu_2Sn_{0.5}V_3W_{1.2}Mo_{12}O_{49.6}$ — coated on inert 4.762 mm $Al_2O_3$ was prepared using known procedures, and 20 cc of this catalyst was placed on each support tray. Where more than one tray was used, the distance between the trays was approximately 38.1 mm. The depth of the catalyst on the trays was 35.5 to 36.5 mm. The fixed-bed catalyst was supported by a stainless steel screen of approximately 1.00 mm opening, which in turn was supported by the trays.

The reactor was fed a stream of propylene, air and water in the mole ratio of 1/10/6. The reaction was run at atmospheric pressure while varying the temperature. The contact time was about 5.0 seconds at a gas velocity of 7 to 17 cm/sec.

Comparative Example A was run without the installation of the fixed-bed catalyst.

Example 1 shows the effect of the uppermost first tray in the catalyst reaction zone being the fixed-bed type.

Examples 2 and 3 show two fixed-bed trays being used, and the effect of varying the reaction temperature. The data for these experiments are shown in Table I. Single pass yield is defined as the moles of product obtained over the moles of propylene in the feed. The temperature shown in that of the middle of the reactor.

TABLE I
Acrylic Acid Production

| Example | No. of Fixed Bed Trays | Temperature °C | % Single Pass Yield | |
|---|---|---|---|---|
| | | | Acrylic Acid | Acrolein |
| Comp. A | 0 | 332 | 28.7 | 58.0 |
| 1 | 1 | 332 | 51.1 | 26.9 |
| 2 | 2 | 332 | 64.1 | 9.7 |
| 3 | 2 | 349 | 69.8 | 3.1 |

It can be seen from these results that the present invention achieves high acrylic acid production in one reactor with combination fixed and fluid beds. The results are dramatic when compared to a blend of fluid-bed catalyst alone.

Comparative Example B and Examples 4 to 6

The same reactor and feed was used for Comparative Example B and Examples 4 to 6. However, the composition of the fluid catalyst was changed. A first fluid catalyst of 90% of a 50% $K_{0.1}Ni_{2.5}Co_{4.5}Fe_3Bi_1P_{0.5}Mo_{12}O_{50.3}$ — 50% $SiO_2$ was used, with the second fluid-bed catalyst remaining the same. The number of trays was varied from 1 to 6.

In Experiments 5 and 6 the upper fluid bed containing the fixed-bed zones was held at 360 to 377°C.

TABLE II
Acrylic Acid Production

| Example | Fixed Bed Trays | Temperature °C | % Single Pass Yield | |
|---|---|---|---|---|
| | | | Acrylic Acid | Acrolein |
| Comp. B | 0 | 349 | 25.5 | 60.3 |
| 4 | 1 | 349 | 42.4 | 41.8 |
| 5 | 3 | 340 | 59.2 | 5.7 |
| 6 | 6 | 332 | 62.1 | 0.6 |

As can be seen by this table, the number of fixed-bed zones increases the desired reaction. Also, the present invention allows the reactor to be controlled non-isothermally for potentially better results.

## 0 010 206

**Claims**

1. A process for the oxidation of propylene to acrylic acid and of isobutylene to methacrylic acid characterized in that the gas phase reactions of the olefin to the aldehyde and aldehyde to the acid occur within a single fluid-bed reactor having a fluid-bed catalyst suitable for converting olefins to aldehydes, and having within said fluid bed one or more fixed bed catalysts suitable for the conversion of aldehydes to acids.

2. A process according to claim 1 characterized in that propylene is oxidized to acrylic acid.

3. A process according to claim 1 characterized in that isobutylene is oxidized to methacrylic acid.

**Patentansprüche**

1. Verfahren zur Oxydation von Propylen zu Acrylsäure und von isobutylen zu Methacrylsäure, dadurch gekennzeichnet, daß die Reaktionen des Olefins zum Aldehyd und des Aldehyds zur Säure in der Gasphase in einem einzigen Fließbett-Reaktor stattfinden, der einen zum Umsetzen von Olefinen zu Aldehyden geeigneten Fließbett-Katalysator und im Fließbett einen oder mehrere, ein festes Bett bildende, zum Umsetzen der Aldehyde zu Säuren geeignete Katalysatoren enthält.

2. Verfahren nach Anspruch 1, dadurch gekennzeichnet daß Propylene zu Acrylsäure oxidiert wird.

3. Verfahren nach Anspruch 1, dadurch gekennzeichnet, daß Isobutylen zu Methacrylsäure oxidiert wird.

**Revendications**

1. Procédé d'oxydation de propylène à l'acide acrylique et d'isobutylène à l'acide méthacrylique, caractérisé en ce que la réaction en phase gazeuse de l'oléfine à l'aldéhyde et de l'aldéhyde à l'acide est effectuée dans un réacteur à lit fluidisé unique et approprié pour la transformation d'oléfines en aldéhydes et comprenant dans ce lit fluidisé un ou deux catalyseurs à lit fixe appropriés pour la transformation d'aldéhydes en acides.

2. Procédé suivant la revendication 1, caractérisé en ce que du propylène est oxydé à l'acide acrylique.

3. Procédé suivant la revendication 1, caractérisé en ce que de l'isobutylène est oxydé à l'acide méthacrylique.

5

**0 010 206**